# EUROPEAN PATENT APPLICATION

(11) **EP 4 120 283 A2**
(43) Date of publication of application: **18.01.2023**
(21) Application number: 22185093.6
(22) Date of filing: 15.07.2022
(51) Int. Cl.: G16H 20/17, G16H 40/63

(54) **SECONDARY METHODS OF COMMUNICATING WITH A DRUG DELIVERY DEVICE**

(30) Priority: 16.07.2021 US 202163222499 P
(71) Applicant: Insulet Corporation, Acton MA 01720 (US)
(72) Inventor: NARAYANASWAMI, Rangarajan, Weston (US)
(74) Representative: Peterreins Schley

(57) **Abstract**

The disclosed embodiments are directed to secondary methods of communicating with a drug delivery device when the primary method is unavailable. Secondary methods include the use of a pattern of taps on the housing of the drug delivery device to convey authentication information and commands to the drug delivery device. Feedback confirming the interpretation of the pattern of taps may be provided to the user via a vibration, sound, or blinking lights.

## Description

### BACKGROUND

Conventional automatic drug delivery systems may deliver a liquid drug, for example, insulin, over the course of a day via one or more basal dosages and/or one or more bolus dosages administered by a drug delivery device. The drug delivery device may be, for example, a pre-filled, disposable device that is worn on the body of the user. Control of the drug delivery device to deliver required basal and/or bolus dosages of the liquid drug may be accomplished in accordance with a dosing algorithm which configures the size and timing of both basal and bolus dosages of the liquid drug.

Users may have the means to control certain aspects of the operation of the drug delivery device through the use of an application installed on a personal computing device (e.g., a smartphone or a smartwatch). For example, a person suffering from Type I diabetes may wish to instruct the drug delivery device to deliver a post-prandial bolus dose of insulin or may wish to temporarily alter the delivery of basal doses of insulin.

Typically, a personal computing device may communicate with the drug delivery device via a wireless connection, for example, a Bluetooth connection. However, an emergency situation may arise in the event that the user is unable to communicate with the drug delivery device via their personal computing device. For example, the user may have unwittingly left the personal computing device at home when leaving the house or the personal computing device may have run out of power. In such an event, the delivery of the liquid drug to the user may be compromised. For example, in the scenario wherein a Type I diabetic is using a drug delivery device to deliver insulin, the inability to instruct the drug delivery device to administer a post-prandial bolus dose of insulin or the inability to be able to instruct the drug delivery device to temporarily reduce the administration of basal doses of insulin may elevate to an emergency situation.

Therefore, it would be desirable to have a secondary means of communicating with the drug delivery device to maintain the ability to alter the default operation of the device.

### DEFINITIONS

As used herein, the term *"****liquid drug*"** should be interpreted to include any drug in liquid form capable of being administered by a drug delivery device via a subcutaneous cannula, including insulin or co-formulations of two or more of GLP-1, pramlintide, and insulin.

As used herein, the terms **"*user*"*****,** "**person**"* and *"**patient**"* are used interchangeably and are meant users of a drug delivery system having features as described herein.

### SUMMARY

This Summary is provided to introduce a selection of concepts in a simplified form that are further described below in the Detailed Description section. This Summary is not intended to identify key or essential features of the claimed subject matter, nor is it intended as an aid in determining the scope of the claimed subject matter.

Disclosed herein are methods for providing a secondary means of communicating with a drug delivery device in the event that the primary means, for example, an application running on a personal computing device in wireless communication with the drug delivery device, fails or is otherwise unable to effectively communicate the desires of the user to the drug delivery device.

In one aspect of the invention, the user may issue commands to the drug delivery device via a series of tapping motions on one or more portions of a housing of the drug delivery device. The drug delivery device may be provided with a vibration sensor, for example, an accelerometer, that detects the tapping motions and interprets a pattern of tapping motions indicating an authentication phrase or a command to alter the behavior of the drug delivery device. Feedback to the user regarding the input of authentication phrase and/or commands may be via a tactile signal provided by a vibration transducer, an audible signal provided by an audio transducer or a visual signal provided by one or more lights visible through the housing of the device.

In a second aspect of the invention, the user may be provided with a display device which may be used for feedback to inform the user of the current operation of the drug delivery device. The display device may wirelessly communicate with the drug delivery device via, for example, a near-field communications interface when the display device is brought in close proximity to the drug delivery device. In another embodiment, the display device may be provided with a fingerprint sensor which may be used to authenticate a user to the drug delivery device.

In a third aspect of the invention, the drug delivery device may be provided with a means for temporarily forming a wireless connection to a "rescue" personal computing device, which may be, for example, a personal computing device of another person who is willing to allow the user to temporarily connect the rescue personal computing device to the drug delivery device to issue commands. The wireless connection may be provided via a Bluetooth connection between the rescue personal computing device and the drug delivery device.

Preferred embodiments of the invention may comprise any combination of the various embodiments or aspects of the invention discussed above.

### BRIEF DESCRIPTION OF THE DRAWINGS

To easily identify the discussion of any particular element, the most significant digit or digits in a reference number refer to the figure number in which that element is first introduced.
**FIG. 1** is a block diagram of an exemplary drug delivery system of the type on which the present invention may be implemented.
**FIG. 2** is a block diagram showing the process whereby the drug delivery device is woken up and placed in a mode wherein a command may be entered by tapping on a housing of the drug delivery device.
**FIG. 3** is an illustration of the Roman alphabet which, in one embodiment, is used for the entry of an authentication phrase and commands.
**FIG. 4** shows a first embodiment of an optional display which may be used with the drug delivery device.
**FIG. 5** shows a second embodiment of the display of **FIG. 4** which also includes a fingerprint sensor which may be used for authenticating a user to the drug delivery device.
**FIG. 6** is a flowchart showing the process by which the user may wake up the device, enter the authentication phrase and thereafter enter commands to the device using a tapping motion on a housing of the device.
**FIG. 7** is a flowchart showing a process by which the user may enter commands using a series of tapping motions on a housing of the device.

### DETAILED DESCRIPTION

Devices and methods in accordance with the present disclosure will now be described more fully with reference to the accompanying drawings, where one or more embodiments or various aspects of the invention are shown. The methods may be embodied in many different forms and are not to be construed as being limited to the embodiments set forth herein. Instead, these embodiments are provided so the disclosure will be thorough and complete, and will fully convey the scope of the systems and methods to those skilled in the art. Each of the methods disclosed herein provides one or more advantages over conventional systems and methods.

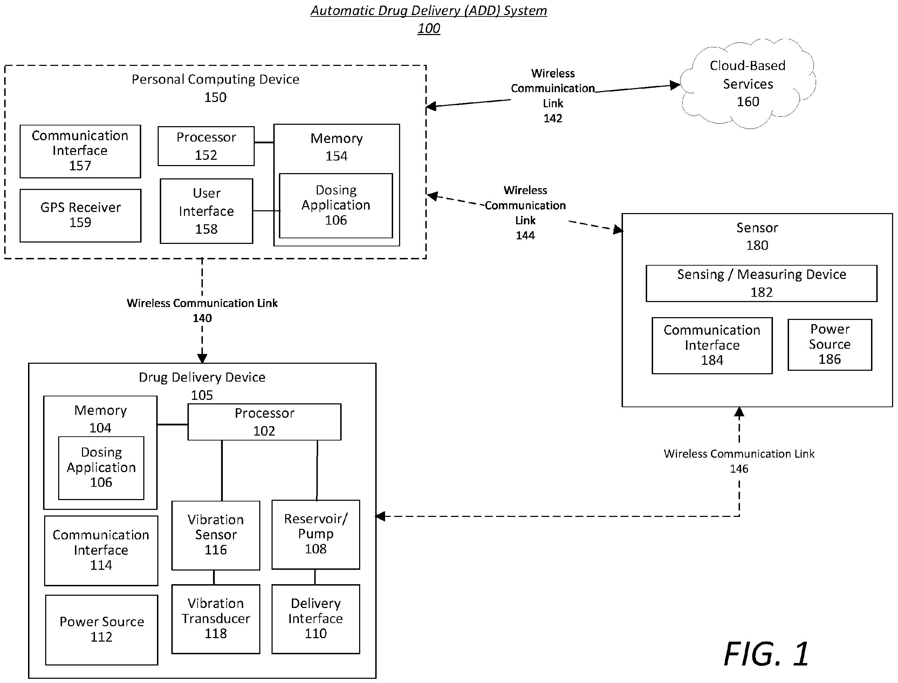

**FIG. 1** is a block diagram showing automatic drug delivery (ADD) system 100 of the type in which various embodiments of the present invention may be implemented. The ADD system 100 includes personal computing device 150 wirelessly connected to drug delivery device 105 via wireless connection 140. The ADD system 100 includes a dosing application 106 for calculating the quantity and timing of the delivery of a liquid drug, for example, basal and bolus doses of insulin, and for controlling the mechanical aspects of drug delivery device 105 responsible for the actual delivery of the liquid drug to the user. The dosing application 106 may implement the various novel embodiments of the invention described in the Summary above and in more detail below.

In a primary embodiment of the invention, the dosing application 106 may be implemented as software executing on a processor in drug delivery device 105 as part of ADD system 100. Drug delivery device 105 may be, for example, a wearable drug delivery device which is discreetly disposed on the body of the user and held in place on the user's skin by an adhesive. Alternatively, drug delivery device 105 may be, for example, a drug delivery device carried by a user (e.g., on a belt or in a pocket) and having an infusion set with tubing connecting the drug delivery device 105 to a cannula that penetrates the skin of the user.

Drug delivery device 105 may include a processor 102 in communication with memory 104 containing dosing application 106 which is executed by processor 102. In accordance with dosing application 106, processor 102 may control one or more reservoirs/pumps 108 suitable for delivering the liquid drug to the user via delivery interface 110 which may be, for example, a subcutaneous cannula extending from one or more housings of drug delivery device 105 and into the body of the user.

Drug delivery device 105 may further include a wireless communication interface 114. Wireless communication interface 114 may comprise, for example, one or more of a Bluetooth interface, a WiFi interface, a near-field interface, an RFID interface or any other wireless interface now known or later developed. Drug delivery device 105 may further include a vibration sensor 116, which may be used to sense tapping patterns on a housing of drug delivery device 105. Vibration sensor 116 may be, for example, a 3-axis accelerometer which can operate in low-power and high-power modes. Drug delivery device 105 may further include a vibration transducer 118 which may be used to send tactile vibrational feedback to the user. Vibration transducer 118 could be of a type that is commonly used in cellular phones, for example, an eccentrically mounted electric motor or a vibrating motor coin. Alternatively (or additionally), feedback to the user may be provided via an audio transducer (not shown) or one or more lights (e.g., LEDs, not shown) which may be visible through a housing of the device 105. The overall drug delivery device 105 may be powered by power source 112, which may be, for example, a battery or a power harvesting apparatus.

ADD system 100 may further include a sensor 180 which comprising a sensing / measuring device 182 which may be, for example, a continuous glucose monitor (CGM). Like drug delivery device 105, sensor 180 may also be a wearable device disposed on the body of the user. Sensor 180 may include power source 186 and a wireless communication interface 184.

Drug delivery device 105 and sensor 180 may communicate with each other via wireless communication link 146. Sensor 180 may provide periodic readings of the blood glucose level of the user to drug delivery device 105 for use by the dosing application 106 via wireless communication link 146. For example, sensor 180, in one embodiment, may provide blood glucose level readings to drug delivery device 105 every 5 minutes. Other intervals for reporting the blood glucose levels of the user are within the scope of the invention. In some embodiments of the present invention, the periodic readings of the blood glucose level of the user may be used, as described below, to modify delivery of the liquid drug.

In alternate embodiments, dosing application 106 may execute on a personal computing device 150 instead of on drug delivery device 105 and may communicate the dosing requirements of the liquid drug to drug delivery device 105 via wireless communication link 140. Personal computing device 150 may be configured with a processor 152 and a memory 154 containing software embodying the dosing application 106 for execution on processor 152. Personal computing device 150 may be further configured with a user interface 158 which may be used by the dosing application 106 to enable interaction with a user.

In some embodiments, personal computing device 150 may comprise, for example, a smartphone, a tablet device, a smartwatch, a dedicated personal diabetes manager, or any other personal mobile computing device capable of executing dosing application 106 and communicating with drug delivery device 105, cloud-based services 160 and sensor 180 via any well-known wireless communication protocol.

Dosing application 106 may have a user interface 158 which may be used, for example, to initialize operation of drug delivery device 105, to initiate administration of bolus doses of the liquid drug or to alter the parameters of the administration of basal doses of the liquid drug. Additionally, user interface 158 may be used to provide feedback to the user regarding the delivery of the liquid drug and the sensed readings from sensor 180. In some embodiments, the sensed readings may be received from sensor 180 directly via communication link 144 or indirectly via drug delivery device 105 via wireless communication link 140.

In alternative embodiments wherein no sensor 180 is used, the user may manually take readings and enter the readings directly to dosing application 106 via user interface 158. The readings may thereafter be transmitted to drug delivery device 105 via wireless communication link 140 for use by dosing application 106.

In some embodiments, the functionality of dosing application 106 may be split between personal computing device 150 and drug delivery device 105 in any convenient manner and separate portions of dosing application 106 executing on personal computing device 150 and on drug delivery device 105 may communicate via wireless link 140.

Dosing application 106 may further communicate with cloud-based services 160 via communication link 142. In alternative embodiments of the invention, the dosing application 106 may be implemented in a cloud-based service 160 and accessed via wireless communication link 142 with the user's personal computing device 150. Further, cloud-based services 160 may provide a data store for storage of the user's past use of the liquid drug for use by dosing application 106.

In one embodiment, dosing algorithm 106 may control the delivery of basal doses of insulin to the user at periodic intervals based on a specified basal rate. Dosing application 106 may comprise an algorithm which periodically cycles. For example, a cycle may comprise a 5-minute period during which a new blood glucose level reading may be received from sensor 180. The dosing application 106 may determine, in response to each additional blood glucose level reading, if additional insulin is required above and beyond the insulin that is indicated by a baseline basal rate and may control the delivery of the additional insulin during the current cycle. This additional insulin may be delivered as micro-boluses, which are insulin amounts above the baseline basal rate. Accordingly, the basal rate may not change, but additional insulin may be delivered as micro-boluses to address excursions in the user's blood glucose levels.

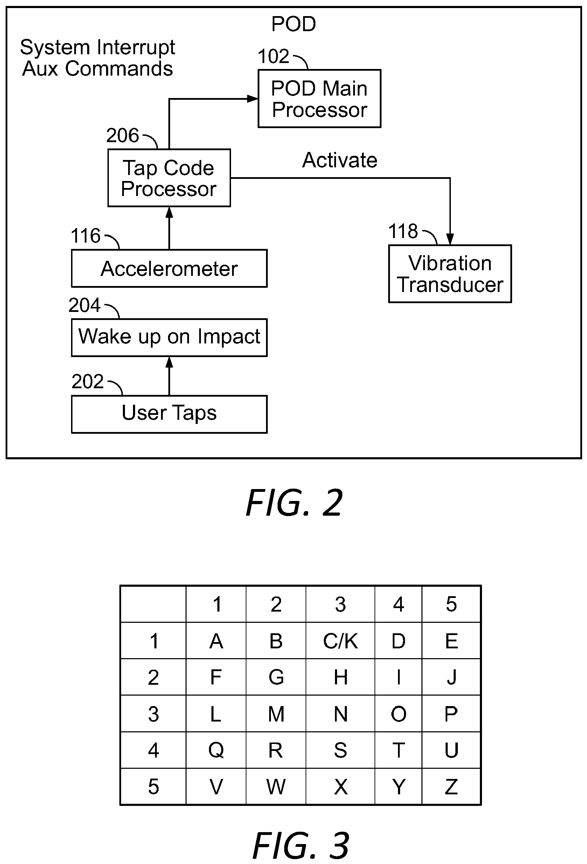

In a first aspect of the invention, as illustrated in FIG. 2, the user may communicate with drug delivery device 105 via a series of taps of various patterns, which may be accomplished by the user tapping a housing (not shown) of the drug delivery device 105 with a finger in a purposeful manner. As previously mentioned, drug delivery device 105 is preferably provided with a vibration sensor 116 which may be, for example, a three-axis accelerometer or any other such similar device. Preferably, the vibration sensor 116 will be held in a low-power state to conserve power source 112 and may be woken up by a single purposeful tap 202 on the housing of drug delivery device 105. Once vibration sensor 116 is awoken at 204, it may be shifted to a high-power state, wherein the tap code processor 206 is listening for specific patterns of taps. Tap code processor 206 may be implemented independently of the main processor 102 of drug delivery device 105, or, alternatively, processor 102 may also process the tap codes under control of dosing application 106, as part of dosing application 106 or as other software stored in memory 104.

Once vibration sensor 116 has been moved to the high-power state, tap code processor 206 may listen for specific patterns of taps indicating entry of an authentication phrase or a command. For example, once vibration sensor 116 is awoken by a single purposeful tap, the user may tap a specific pattern (e.g., three purposeful taps in quick succession) to place the tap code processor 206 in listening mode, in which tap code processor 206 is listening for specific commands to be entered via other tapping patterns.

Drug delivery device 105 may also be provided with a vibration transducer 118 which may be used to provide feedback to the user. Alternatively, drug delivery device 105 may be provided with an audio transducer (not shown) or a series of lights (not shown) which may be visible through a housing of the device to provide feedback to the user. Feedback to the user may be provided after each step of the communication process, for example, as a simple pattern of vibrations, audible chirps or blinking lights. In a preferred embodiment of the invention, the vibration transducer 118 will be used to provide feedback to the user as tactile feedback is preferred over audio or visual feedback. As an example, the user may have trouble hearing or seeing, or the drug delivery device 105 may be positioned on the user's body in a place that makes it inconvenient to see visual feedback. In other embodiments wherein the drug delivery device is not wearable, the preferred method of feedback may be via an audible signal. In other embodiments, user feedback may be provided by two or more of tactile feedback, audio feedback, or visual feedback. As an example, after each step of the communication process, the user may be provided with a pattern of vibrations, chirps and/or blinking lights, but, as would be realized, any combination of vibrations, chirps or blinks may be used to acknowledge command inputs from the user.

In preferred embodiments of the invention, once the vibration sensor 116 has been placed in a high-power state and tap code processor 206 is ready to receive commands, the user must first provide authentication information to drug delivery device 105 before any other commands can be accepted. In one embodiment, a Roman alphabet, shown in FIG. 3, may be used to enter a short authentication phrase. The Roman alphabet is implemented in a tap code table which has five rows and five columns. Each letter is coded by a pair of numbers indicating its row and column in the table. The pair of numbers is communicated by a first sequence of quick purposeful taps indicating the first number in the pair, a short pause, and a second sequence of quick purposeful taps, indicating the second number in the pair. For example, the letter "S" can be coded as (4, 3), which is communicated to drug delivery device 105 as four quick taps, a short pause, and three quick taps. The user must remember only the sequence "AFLQV" to remember the letter row and column positions to execute the code. The user may then enter an authentication phrase using the series of taps. As would be realized, is preferred that the authentication phrase be short, for example, four letters, to avoid errors which may easily occur when entering letters using the tapping motions.

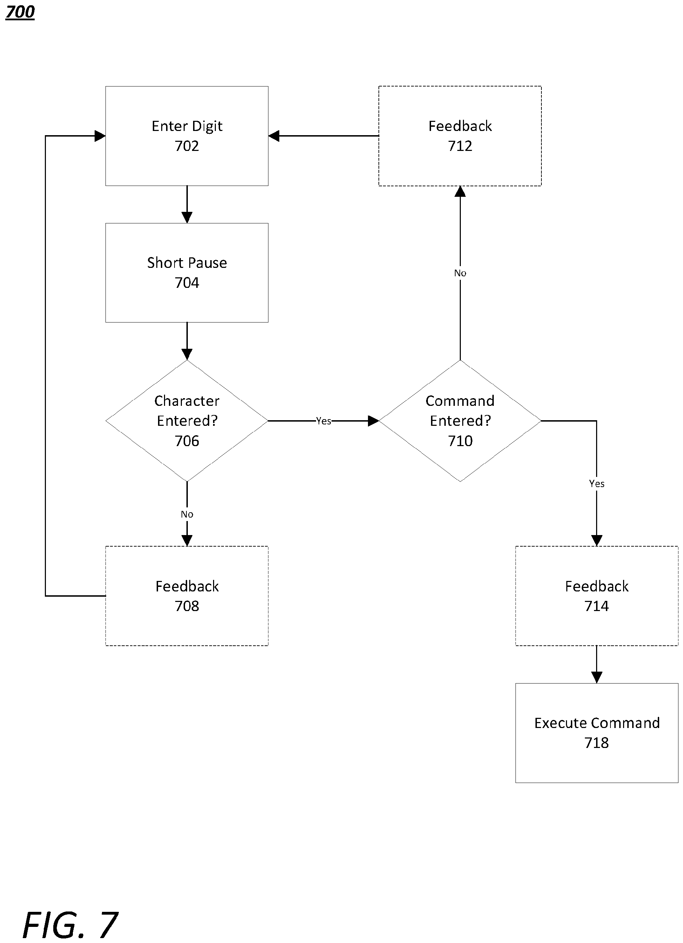

As an example, if the user has set their authentication code as "PSWD", this could be encoded as (3, 5), (4, 3), (5, 2), (1, 4). This could be tapped as a series of purposeful taps as: three taps, a short pause, five taps, a long pause, four taps, a short pause, three taps, a long pause, five taps, a short pause, two taps, a long pause, one tap, a short pause, four taps. The user may be assisted in entering the string of characters by feedback provided by drug delivery device 105, for example, after a short pause the vibration transducer 118 could provide one vibration and, after, a long pause, the vibration sensor 118 could provide two vibrations. In lieu of a long pause, tap code processor 206 may automatically recognize that a complete character or a complete command has been entered (see example below with respect to **FIG. 7**), which would eliminate the need for the long pauses. The feedback makes it easier for the user to determine that tap code processor 206 is correctly interpreting the taps. In alternative embodiments, the user may enter a PIN code as an alternative to a series of characters. The digits of the PIN code could simply be entered by a number of taps corresponding to the digits of the PIN code, followed by a short pause between numbers. Feedback may be provided between the entry of each number, for example, vibration transducer 118 could provide a single vibration after each digit has been entered. In yet other embodiments of the invention, other means of entering authentication information may be provided. For example, the user may have initially set up a predetermined pattern of taps and pauses not corresponding to any characters or numbers to be used as an authentication phrase.

The user may initially indicate the authentication phrase via user interface 158 for dosing application 106, which is executed on personal computing device 150. The user may enter the authentication phrase one time and, each time a new drug delivery device 105 is used, the information regarding the authentication phrase is transferred via wireless communication link 140 to the new drug delivery device 105 during initialization of each new drug delivery device 105.

Once the authentication information has been provided by the user and verified by a portion of dosing application 106 executing on processor 102 of drug delivery device 105, the device may be ready to accept commands. In preferred embodiments of the invention, the user will be able to provide a command indicating to drug delivery device 105 that a bolus dose of the liquid drug should be administered. In addition, the volume of the bolus dose may also be communicated to the drug delivery device. Additionally, in preferred embodiments, the user may wish to deliver a temporary basal dose of the liquid drug and provide a temporary basal volume. Other commands may also be made available, depending on the liquid drug being administered and the particular mode that dosing application 106 is executing.

Examples will now be provided wherein commands for administration of bolus and basal doses of the liquid drug are illustrated. As would be realized, the actual set of commands that may be recognized by drug delivery device 105 may include commands other than those specifically discussed herein.

If the user wishes the drug delivery device 105 to administer a bolus dose of the liquid drug, this can be communicated with a chosen two letter code. For example, if the command is "give bolus" the user may enter the two-character command "GB". Using the Roman alphabet of FIG. 3, the command "GB" can be entered as (2, 2), (1, 2). In this case, the tapping sequence would be two taps, short pause [one vibration provided], two taps, [character automatically detected and two vibrations provided], one tap, short pause [one vibration provided], two taps [character and command automatically detected, three vibrations provided]. Feedback may be provided, as previously discussed, after each digit is entered, after a character is recognized and/or after a command is recognized, as in the example just provided. Alternatively, any combination of vibrations providing user feedback may be utilized. The volume of the bolus dose can be specified as a number of units of insulin and tapped out as a single digit. For example, if two units of insulin is requested, the user may provide two taps. The acceptance of the number of bolus units may be acknowledged by providing vibrational feedback, for example, two vibrations.

In another example, the user may wish to request that the drug delivery device 105 provide a temporary basal delivery. This could be encoded as a two-character command "TB" which is encoded as (4, 4), (1, 2) and tapped out as four taps, short pause [one vibration provided], four taps [character automatically detected and two vibrations provided], one tap, short pause [one vibration provided], two taps [character and commend automatically detected and three vibrations provided]. As previously, feedback may be provided after each digit is entered, after each character is recognized and/or after the entire command is recognized. The temporary basal volume can be a fractional number. This may be tapped as two numbers, with the first number being the numerator of the fraction and the second number being the denominator of the fraction. For example, 0.5 units can be encoded as (1, 2) and tapped as one tap, short pause [one vibration provided], two taps [character and command recognized, three vibrations provided]. In a similar manner, 1.0 units could be encoded as (1, 1), 1.5 units could be coded as (3, 2), etc. As previously, feedback may provide after each digit is centered or after the entire dose volume has been entered.

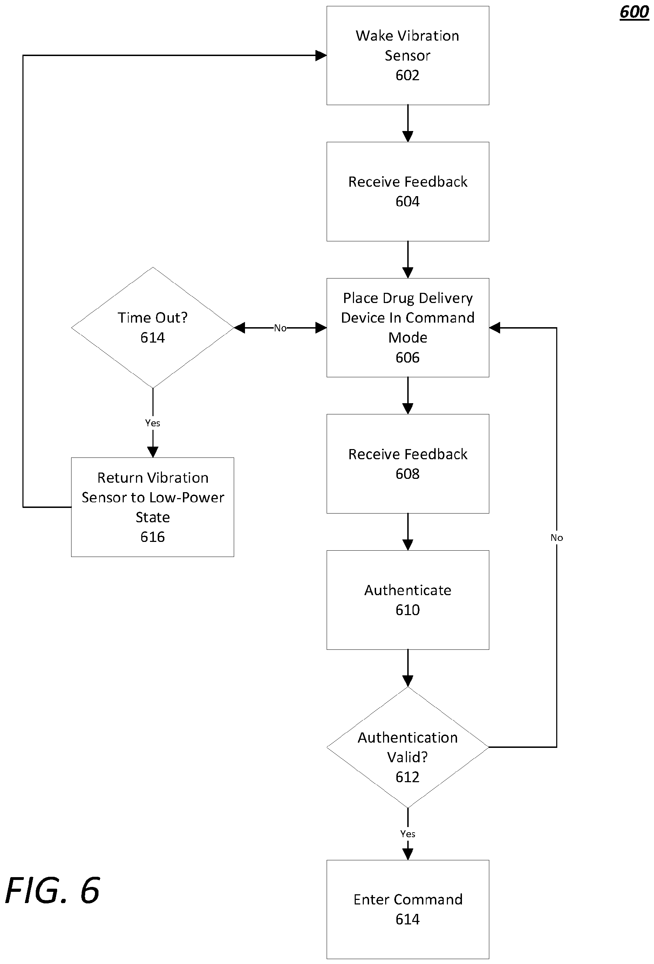

**FIG. 6** shows process 600 by which the user is able to enter a command directly to drug delivery device 105 using a pattern of taps as previously described. At 602, the user provides, for example, a single purposeful tap on a housing of the device to wake the vibration sensor 116. At this point, the vibration sensor 116 moves from a low-power state to a high-power state and is ready to accept further taps. At 604, feedback is provided to the user acknowledging the waking of vibration sensor 116, preferably in the form of vibrations produced by the vibration transducer 118. The feedback may be, for example, a single vibration. At 606, the user provides a pattern of taps at places the drug delivery device 105 in command mode. That is, drug delivery device 105 is ready to receive further commands from the user. The tapped pattern this case may be, for example, three quick successive purposeful taps on a housing of the device. At 606, feedback is provided to the user acknowledging that the drug delivery device 105 is now in command mode. The feedback is preferably in the form of one or more vibrations produced by the vibration transducer 118, for example, a series of three quick successive vibrations may be used. At 610, the user is required to enter the authentication phrase. At 612, if the authentication phrase is valid, control moves to 614 where the user may enter commands. At 612, if the authentication phrase is invalid, control returns to at 606, where drug delivery device 105 will again wait for the user to place the drug delivery device 105 in command mode. If no entries are made after a certain period of time, at 614, the tap code processor 206 will time out and, at 616, vibration sensor 116 will be returned to a low-power mode state and process 600 and will have to be restarted at 602 to allow the user to enter commands.

**FIG. 7** shows process 700 which may be used by the user to enter either the authentication phrase from process block 610 of **FIG. 6** or a command from process block 614 of **FIG. 6**. Note that process 700 is exemplary in nature and assumes that the scheme described above is used to enter commands using the Roman alphabet. Process 700 is exemplary only and specific to the entry of commands using the Roman alphabet and would change if other schemes for entering the commands are used. At 702, the user enters a digit by tapping a number of times corresponding to the desired digit and, at 704, provides a short pause indicating that the entry of the digit is complete. At 706, if the drug delivery device 105 does not recognize that a full character has been entered (i.e., both digits of the pair), feedback is optionally provided, preferably in the form of a single vibration indicating that only the first digit of the pair of digits indicating the character has been entered. Control then returns to 702, where the user enters another digit. If, at 706, drug delivery device 105 recognizes that a character has been entered (i.e. both digits in the pair of digits have been entered), control passes to 710, where drug delivery device 105 determines if an entire command has been entered. If an entire command has not been entered (i.e. drug delivery device 105 expects more characters to complete the command), feedback is optionally provided at 712, preferably in the form of two vibrations, indicating that a character has been entered, but not an entire command. Control then returns to 702 where the user is expected to enter the next digit. At 710, if drug delivery device 105 determines that a command has been entered, control proceeds to 714 where feedback may be optionally provided, preferably in the form of three vibrations indicating that a full command has been recognized. Control then passes to 718 where drug delivery device 105 executes the indicated command.

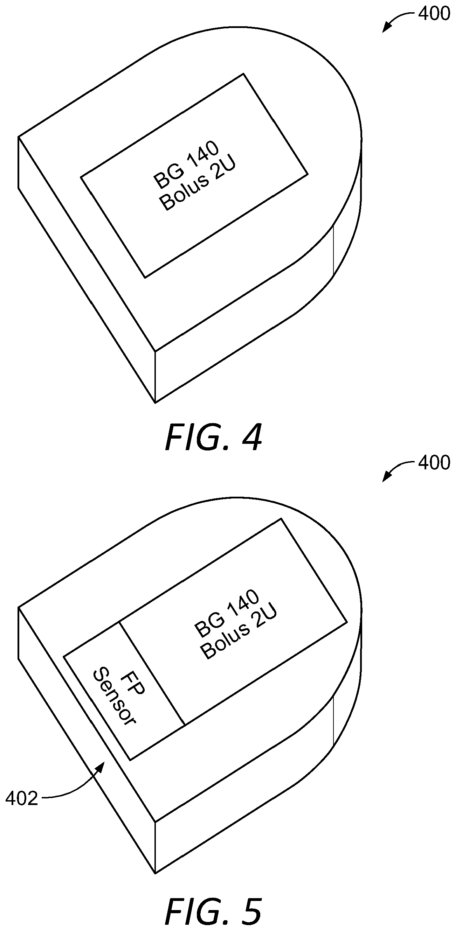

In another aspect of the invention, the user may be provided a portable display 400, shown in various embodiments in **FIG. 4** and **FIG. 5**, which can either be attached to the drug delivery device 105 (e.g., via a suction fit or snap fit) or which may be held in close proximity to the drug delivery device 105. In a preferred embodiment of the invention, display 400 and drug delivery device 105 may communicate with each other via a near-field interface, an RFID interface, a Bluetooth connection, a Wi-Fi connection or by any other known means whereby devices brought in close proximity to each other may be automatically triggered to wirelessly communicate. In preferred embodiments, display 400 will have a power supply independent of the power supply of drug delivery device 105.

In preferred embodiments, display 400 may display any requested parameters of drug delivery device 105 or dosing application 106. For example, as shown in **FIG. 4** and **FIG. 5**, display 400 is showing the current blood glucose value as well as the bolus delivery amount.

In an alternative embodiment, shown in **FIG. 5**, display 400 may also be provided with a fingerprint sensor 402 which may be used to authenticate the user to the drug delivery device 105 in lieu of the user entering the authentication phrase as a tapping pattern as previously discussed. Alternatively, fingerprint sensor 402 can be provided as a standalone device without display 400.

In yet another aspect of the invention, the user may instruct the drug delivery device 105 to pair with a new portable computing device to enter commands. In an example scenario, the user is out with friends at a dinner and needs to request the drug delivery device 105 to administer a post-prandial bolus dose of the liquid drug. However, the user's personal computing device 150, which is paired with drug delivery device 105, has run out of power, and the user has no way to inform the drug delivery device that is time to administer the bolus dose. In such a case, the user may borrow the personal computing device of a friend and may use that device as a "rescue device" to communicate the required command to drug delivery device 105.

The rescue device may communicate with drug delivery device 105 using any one of several different methods. For example, in one embodiment, the user may place drug delivery device 105 in command mode as previously discussed by waking up vibration sensor 116 and indicating to tap code processor 206 that a command is forthcoming. The user may then enter the authentication phrase as previously discussed. Thereafter, the user may enter a command indicating that the new device is to be paired with the drug delivery device 105. In certain embodiments the user can enter a two letter command, for example, "NP" (i.e. new personal computing device) which may be coded as (3, 3), (3, 5). This command may open the Bluetooth connection on the drug delivery device 105 and allow the rescue device to connect to the drug delivery device 105 via the Bluetooth interface. The user can then use the rescue device to control the drug delivery device 105. If information from the previous personal computing device 150 is stored in the cloud, it can be migrated to the rescue device. In certain embodiments, it may be necessary to install dosing application 106 on the rescue device such as to enable communication with drug delivery device 105.

In an alternative embodiment, and, in particular, if the drug delivery device 105 is provided with a Wi-Fi interface, the user may instruct the drug delivery device 105, via a series of taps, as previously discussed, to establish a Wi-Fi connection with the rescue device or to broadcast a Wi-Fi signal which will allow the user to connect by specifying a particular IP address in a web browser on the rescue device. This will cause a rescue device to have access to a simple webpage broadcast by the drug delivery device 105 which may be used for entry of one or more simple commands to be communicated to drug delivery device 105. For example, the user could enter the command "WF" to indicate that the rescue device wishes to connect via Wi-Fi. In alternative embodiments, should the user be successful in accessing a webpage generated by drug delivery device 105 using a browser in the rescue device, the user may enter the authentication phrase using the rescue device in lieu of using the combination of taps as described above.

This embodiment has the advantage that the rescue device does not need to download and install a specific application to communicate with drug delivery device 105, but, instead, may communicate via a standard web browser. This is especially useful in areas where a connection to the Internet, which may be required to download an application, may not be available. It may also be possible for drug delivery device 105 to serve a webpage using communication interfaces other than Wi-Fi. For example, the browser may connect to the webpage generated by drug delivery device 105 via a Bluetooth or near-field connection.

The foregoing has been presented for the purposes of illustration and description. It is not intended to be exhaustive or to limit the present disclosure to the exemplary embodiments disclosed herein. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

Although the present invention is defined in the attached claims, it should be understood that the present Invention can also (alternatively) be defined in accordance with the following embodiments:
1. A drug delivery device comprising:
   a housing;
   a processor, disposed in the housing
   a vibration sensor, disposed in the housing; and
   software, which, when executed by the processor, performs the functions of:
      receiving signals from the vibration sensor indicating a pattern of taps on the housing of the device;
      interpreting the pattern of taps as a command; and
      executing the command indicated by the pattern of taps.
2. The device of embodiment 1 wherein the software performs the further function of:
   providing feedback regarding the analysis of the pattern of taps.
3. The device of one of the preceding embodiments wherein the feedback is provided using one or more of a vibration transducer, an audio transducer or one or more visible indicators.
4. The device of one of the preceding embodiments wherein entry of an authentication phrase indicated by a particular pattern of taps is required before any commands may be entered.
5. The device of one of the preceding embodiments wherein the authentication phrase comprises one or more patterns indicating digits of a PIN number separated by short pauses.
6. The device of one of the preceding embodiments wherein the pattern of taps indicating the authentication phrase may be specified using an application installed on a personal computing device and communicated to the drug delivery device via a wireless interface.
7. The device of one of the preceding embodiments wherein the pattern of taps may include patterns indicating one or more characters in accordance with a predetermined table of characters.
8. The device of one of the preceding embodiments wherein characters are expressed by patterns of taps indicating a row number and a column number of the table separated by a short pause, the row and column number indicating a character positioned at the specified row and column of the table.
9. The device of one of the preceding embodiments, the device further comprising:
   one or more reservoirs containing one or more liquid drugs; and
   one or more pumps associated with the one or more respective reservoirs;
   wherein the software is further configured to:
      analyze patterns of taps representing commands indicating a particular delivery of the one or more liquid drugs stored in the one or more reservoirs.
10. The device of one of the preceding embodiments wherein at least one of the one or more liquid drugs is insulin and wherein the software performs the further function of:
   recognizing a pattern of taps representing a command indicating that the device should administer a bolus dose of the insulin.
11. The device of one of the preceding embodiments, the software performing the further function of:
   recognizing a pattern of taps representing a number of whole or fractional units that should be administered as a bolus dose.
12. The device of one of the preceding embodiments wherein at least one of the one or more liquid drugs is insulin and wherein the software performs the further function of:
   recognizing a pattern of taps representing a command indicating a temporary change in a basal dose of the insulin.
13. The device of one of the preceding embodiments, the software performing the further function of:
   recognizing a pattern of taps representing a number of units that should be administered as a basal dose.
14. The device of one of the preceding embodiments wherein the device returns to a default setting for the delivery of basal doses of insulin after a predetermined period of time.
15. The device of one of the preceding embodiments wherein the vibration sensor is maintained in a low-power state and further wherein the vibration center is woken up by a single tap the housing of the device and is moved to a high-power state.
16. The device of one of the preceding embodiments, the software performing the further function of:
   recognizing a pattern of taps indicating that the device is to enter a mode in which commands may be accepted.
17. The device of one of the preceding embodiments, the software performing the further function of:
   recognizing a command indicating that the device should connect to a new personal computing device and should thereafter receive commands from the new personal computing device.
18. The device of one of the preceding embodiments wherein the device connects to the new personal computing device via one of a Bluetooth interface, a Wi-Fi interface and a near-field interface.
19. The device of one of the preceding embodiments wherein the new personal computing device issues commands to the device using a web browser.
20. The device of one of the preceding embodiments wherein the new personal computing device issues commands to the device using an installed application.
21. A display device comprising:
   a display;
   a power supply; and
   a communication interface;
   wherein the display device displays selected parameters from a drug delivery device when brought in close proximity to the drug delivery device.
22. The display device of embodiment 21 wherein the communication interface is selected from a group comprising a Bluetooth interface, a Wi-Fi interface, a near-field interface and an RFID interface.
23. The display device of one of embodiments 21 or 22 wherein the display device is configured to physically connect to a housing of the drug delivery device.
24. The display device of one of embodiments 21-23 further comprising:
   a fingerprint sensor wherein fingerprints captured using the fingerprint sensor are sent to the drug delivery device as a means of authenticating a user to the drug delivery device.

## Claims

1. A drug delivery device comprising:
a housing;
a processor, disposed in the housing
a vibration sensor, disposed in the housing; and
software, which, when executed by the processor, performs the functions of:
receiving signals from the vibration sensor indicating a pattern of taps on the housing of the device;
interpreting the pattern of taps as a command; and
executing the command indicated by the pattern of taps.

2. The device of claim 1 wherein the software performs the further function of:
providing feedback regarding the analysis of the pattern of taps.

3. The device of one of the preceding claims wherein the feedback is provided using one or more of a vibration transducer, an audio transducer or one or more visible indicators.

4. The device of one of the preceding claims wherein entry of an authentication phrase indicated by a particular pattern of taps is required before any commands may be entered.

5. The device of one of the preceding claims wherein the authentication phrase comprises one or more patterns indicating digits of a PIN number separated by short pauses.

6. The device of one of the preceding claims wherein the pattern of taps indicating the authentication phrase may be specified using an application installed on a personal computing device and communicated to the drug delivery device via a wireless interface.

7. The device of one of the preceding claims wherein the pattern of taps may include patterns indicating one or more characters in accordance with a predetermined table of characters.

8. The device of one of the preceding claims wherein characters are expressed by patterns of taps indicating a row number and a column number of the table separated by a short pause, the row and column number indicating a character positioned at the specified row and column of the table.

9. The device of one of the preceding claims, the device further comprising:
one or more reservoirs containing one or more liquid drugs; and
one or more pumps associated with the one or more respective reservoirs;
wherein the software is further configured to:
analyze patterns of taps representing commands indicating a particular delivery of the one or more liquid drugs stored in the one or more reservoirs.

10. The device of one of the preceding claims wherein at least one of the one or more liquid drugs is insulin and wherein the software performs the further function of:
recognizing a pattern of taps representing a command indicating that the device should administer a bolus dose of the insulin.

11. The device of one of the preceding claims, the software performing the further function of:
recognizing a pattern of taps representing a number of whole or fractional units that should be administered as a bolus dose.

12. The device of one of the preceding claims wherein at least one of the one or more liquid drugs is insulin and wherein the software performs the further function of:
recognizing a pattern of taps representing a command indicating a temporary change in a basal dose of the insulin.

13. The device of one of the preceding claims, the software performing the further function of:
recognizing a pattern of taps representing a number of units that should be administered as a basal dose.

14. The device of one of the preceding claims wherein the device returns to a default setting for the delivery of basal doses of insulin after a predetermined period of time.

15. The device of one of the preceding claims wherein the vibration sensor is maintained in a low-power state and further wherein the vibration center is woken up by a single tap the housing of the device and is moved to a high-power state.
